# EUROPEAN PATENT APPLICATION

(11) **EP 4 760 134 A1**
(43) Date of publication of application: **17.06.2026**
(21) Application number: 25223471.1
(22) Date of filing: 15.12.2025
(51) Int. Cl.: F16L 25/00, A61M 39/10, F16L 37/56, F16L 39/02

(54) **HOSE CONNECTOR ASSEMBLY**

(30) Priority: 16.12.2024 US 202463734329 P; 12.12.2025 US 202519417496
(71) Applicant: Hill-Rom Services, Inc., Batesville, IN 47006-9167 (US)
(72) Inventor: KANDASAMY, Rajesh, Batesville, 47006-9167 (US)
(74) Representative: Reddie & Grose LLP

(57) **Abstract**

A hose connector assembly (10) includes a connector housing (12) that defines an interface opening (14) and a hose receiver (16). A latch (18) is operably coupled with the connector housing (12) and is spring-biased to a latched position. A first hose (20) is configured to engage with the hose receiver (16) and supplies fluid to a patient support interface (22) of a patient support apparatus (24). A second hose (26) is configured to engage with the hose receiver (16) and is smaller in diameter than the first hose (20). A data line (30) is configured to engage a data interface of the patient support apparatus (24). A bottom cover (32) extends over the first and second hoses (20, 26) and a portion of the hose receiver (16) and a top cover (34) extends over the bottom cover (32) and at least partially conceals the latch (18). Fluid is configured to pass through the first and second hoses (20, 26) into the connector housing (12) in a substantially linear direction thereby minimizing noise and fluid loss of the connector assembly.

## Description

The present disclosure generally relates to a hose connector assembly, and more particularly to a hose connector assembly for use with a patient support apparatus.

According to one aspect of the present disclosure, a connector assembly for a patient support apparatus includes a connector housing that defines an interface opening and a hose receiver. A latch is operably coupled with the connector housing and is spring-biased to a latched position. First and second hoses are configured to engage with the hose receiver. A bottom cover extends over the first and second hoses and a portion of the hose receiver and a top cover extends over the bottom cover and at least partially conceals the latch. Fluid is configured to pass through the first and second hoses into the connector housing in a substantially linear direction thereby minimizing noise and fluid loss of the connector assembly.

According to another aspect of the present disclosure, a connector assembly includes a connector housing that defines an interface opening and a hose receiver. A latch is operably coupled with the connector housing and is spring-biased to a latched position. A first hose is configured to engage with the hose receiver and supplies fluid to a patient support interface of a patient support apparatus. A second hose is configured to engage with the hose receiver and is smaller in diameter than the first hose. A data line is configured to engage a data interface of the patient support apparatus. A bottom cover extends over the first and second hoses and the hose receiver and a top cover extends over the bottom cover and at least partially conceals the latch. The fluid is configured to pass through the first and second hoses and through the connector housing in a linear direction thereby minimizing noise and fluid loss of the hose connector assembly.

According to another aspect of the present disclosure, a patient support apparatus includes a patient support and a connector assembly. The connector assembly includes a connector housing that defines an interface opening and a hose receiver. A latch is operably coupled with the connector housing and is spring-biased to a latched position. A first hose is configured to engage with the hose receiver and supplies fluid to a patient support interface of the patient support apparatus. A second hose is configured to engage with the hose receiver. A data line is configured to engage a data interface disposed at the patient support. A top cover extends over a bottom cover. The fluid is configured to transition from the first and second hoses to the hose connector assembly in a linear direction thereby minimizing noise and fluid loss of the connector assembly.

The invention will now be further described by way of example with reference to the accompanying drawings, in which:
FIG. 1 is a side perspective view of a patient support apparatus including a hose and hose connector assembly of the present disclosure;
FIG. 2 is a front top perspective view of a hose connector assembly of the present disclosure;
FIG. 3A is a rear top perspective view of a hose connector assembly of the present disclosure;
FIG. 3B is a rear top perspective view of a hose connector assembly of the present disclosure;
FIG. 4 is an enlarged front top perspective view of a hose connector assembly of the present disclosure;
FIG. 5 is a front top exploded perspective view of a hose connector assembly of the present disclosure;
FIG. 6 is a rear top exploded perspective view of a hose connector assembly of the present disclosure;
FIG. 7 a side perspective, cross-sectional view of a hose connector assembly of the present disclosure;
FIG. 8 is a side perspective view of a patient support interface that is configured to receive a hose connector assembly of the present disclosure; and
FIG. 9 a side perspective view of a hose connector assembly of the present disclosure prior to engagement with a patient support interface of a patient support apparatus.

The present illustrated embodiments reside primarily in combinations of method steps and apparatus components related to a hose connector assembly for use with a patient support apparatus. Accordingly, the apparatus components and method steps have been represented, where appropriate, by conventional symbols in the drawings, showing only those specific details that are pertinent to understanding the embodiments of the present disclosure so as not to obscure the disclosure with details that will be readily apparent to those of ordinary skill in the art having the benefit of the description herein. Further, like numerals in the description and drawings represent like elements.

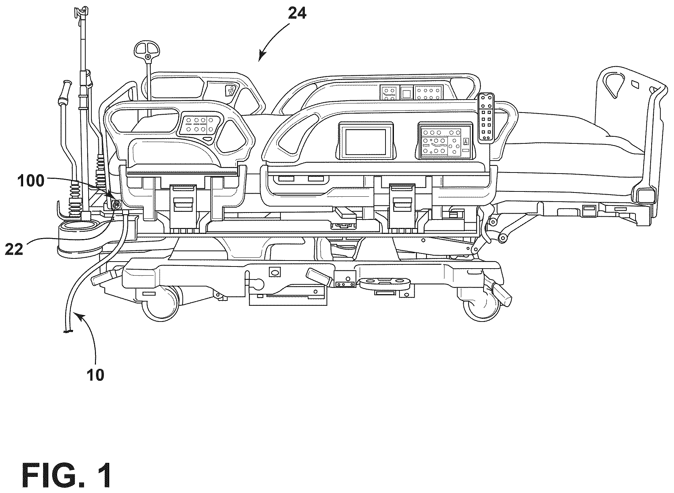

For purposes of description herein, the terms "upper," "lower," "right," "left," "rear," "front," "vertical," "horizontal," and derivatives thereof, shall relate to the disclosure as oriented in FIG. 1. Unless stated otherwise, the term "front" shall refer to a surface closest to an intended viewer, and the term "rear" shall refer to a surface furthest from the intended viewer. However, it is to be understood that the disclosure may assume various alternative orientations, except where expressly specified to the contrary. It is also to be understood that the specific structures and processes illustrated in the attached drawings, and described in the following specification are simply exemplary embodiments of the inventive concepts defined in the appended claims. Hence, specific dimensions and other physical characteristics relating to the embodiments disclosed herein are not to be considered as limiting, unless the claims expressly state otherwise.

The terms "including," "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. An element preceded by "comprises a . . . " does not, without more constraints, preclude the existence of additional identical elements in the process, method, article, or apparatus that comprises the element.

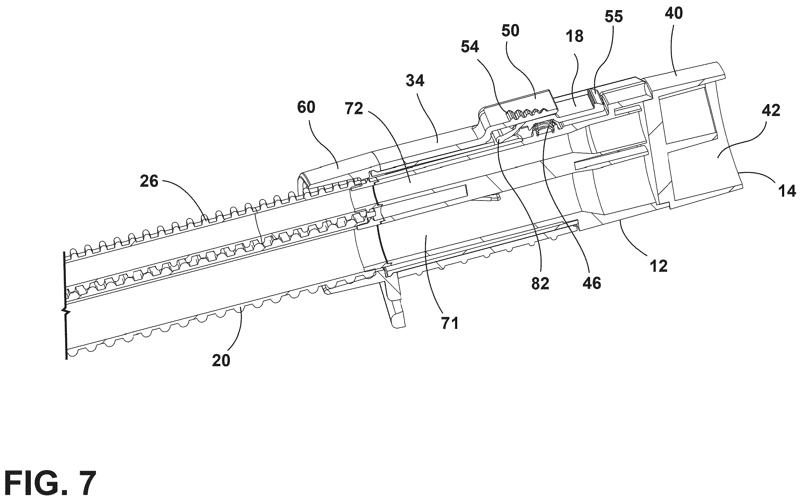

Referring to FIGS. 1-9, reference numeral 10 generally designates a hose connector assembly that includes a connector housing 12 that defines an interface opening 14 and a hose receiver 16. A latch 18 is operably coupled with the connector housing 12 and is spring-biased to a latched position. A first hose 20 is configured to engage with the hose receiver 16 and supplies fluid to a patient support interface 22 of a patient support apparatus 24. A second hose 26 is configured to engage with the hose receiver 16 and is smaller in diameter than the first hose 20. A data line 30 is configured to engage a data interface of the patient support apparatus 24. A bottom cover 32 extends over the first and second hoses 20, 26 and the hose receiver 16 and a top cover 34 extends over the bottom cover 32 and at least partially conceals the latch 18. The fluid is configured to pass through the first and second hoses 20, 26 in a substantially linear direction thereby minimizing noise and fluid loss of the hose connector assembly 10.

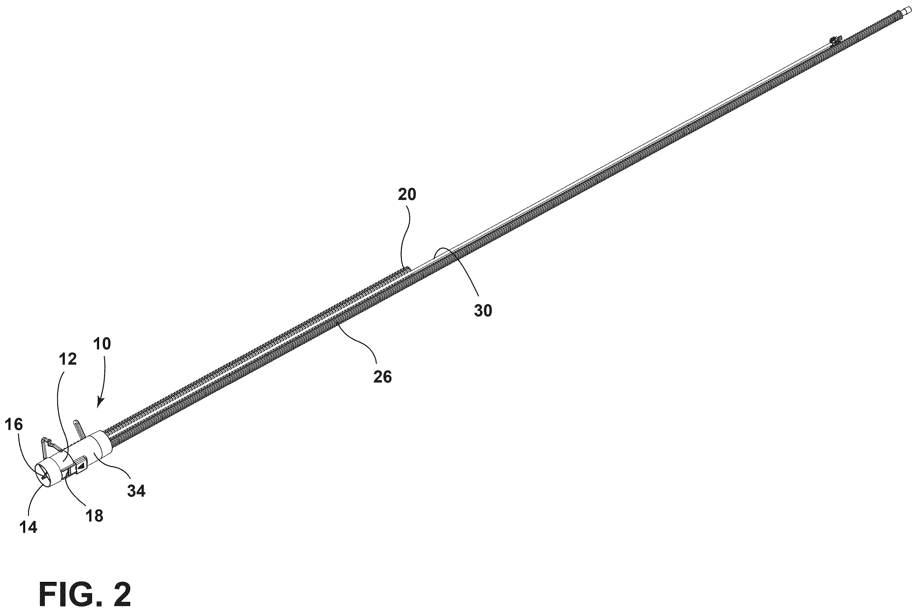

With reference again to FIGS. 1 and 2, the illustrated hose connector assembly 10 is configured for use with a patient support apparatus, such as the patient support apparatus 24 shown in FIG. 1. It will be understood that the patient support apparatus 24 may take on a variety of constructions, including a hospital bed, a surgical table, etc. In addition, the hose connector assembly 10, and specifically the first hose 20, the second hose 26, and the data line 30 may vary in length. In addition, all or a majority of the length of at least one of the first hose 20 and the second hose 26 may be corrugated at a circumference thereof. Both the first hose 20 and the second hose 26 include a hollow hose configuration designed to supply fluid such as oxygen, nitrous oxide, or any other fluid that is being supplied to aid in caring for a patient. The first and second hoses 20, 26 may include a barbed engagement end configured to interface with the connector-housing.

The data line 30 extends adjacent to or between the first hose 20 and the second hose 26. The data line 30 may be integral with or separate from the first hose 20 and/or the second hose 26. The data line 30 may include an electrical interface capable of receiving or sending data from the connector housing 12 to a controller for display on an audio/visual monitor. The data line 30 is configured to relay or receive data related to flowrates, fluid supply, blockages, etc. of the supply of fluid from the first and second hoses 20, 26.

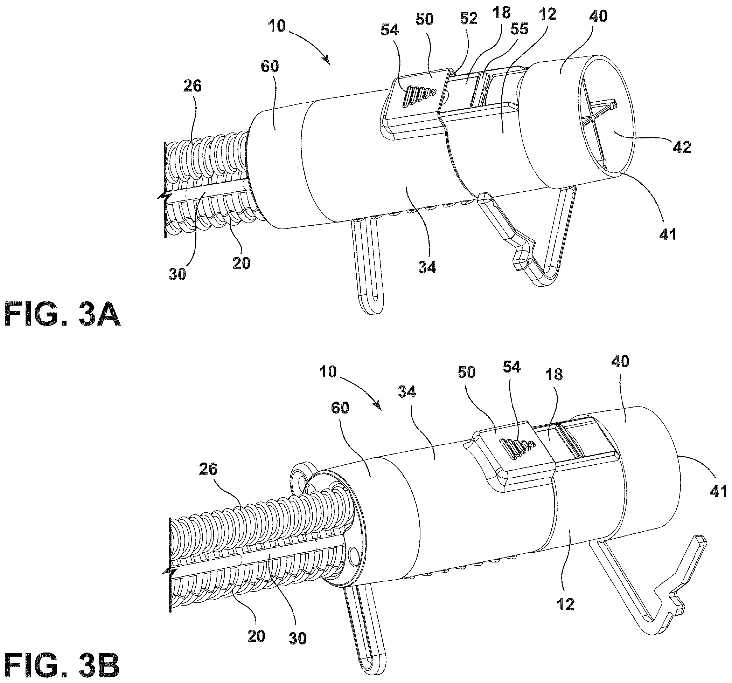

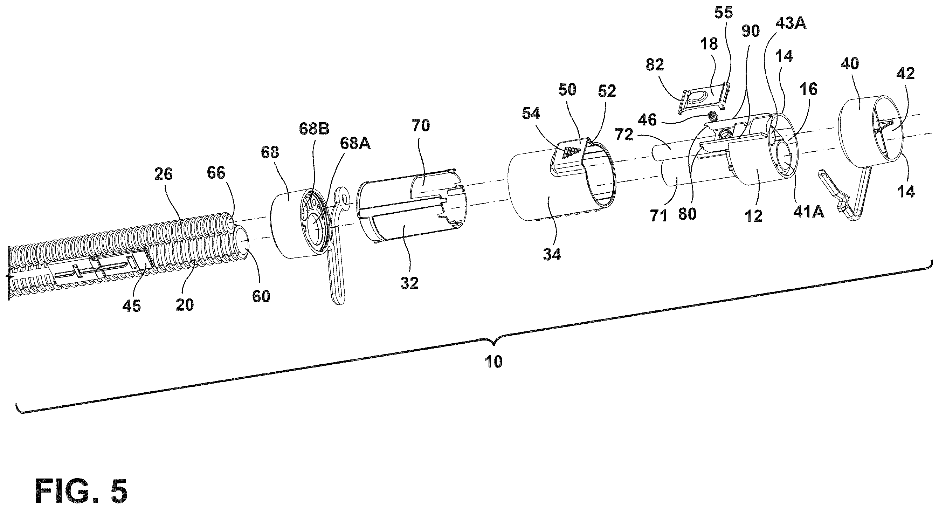

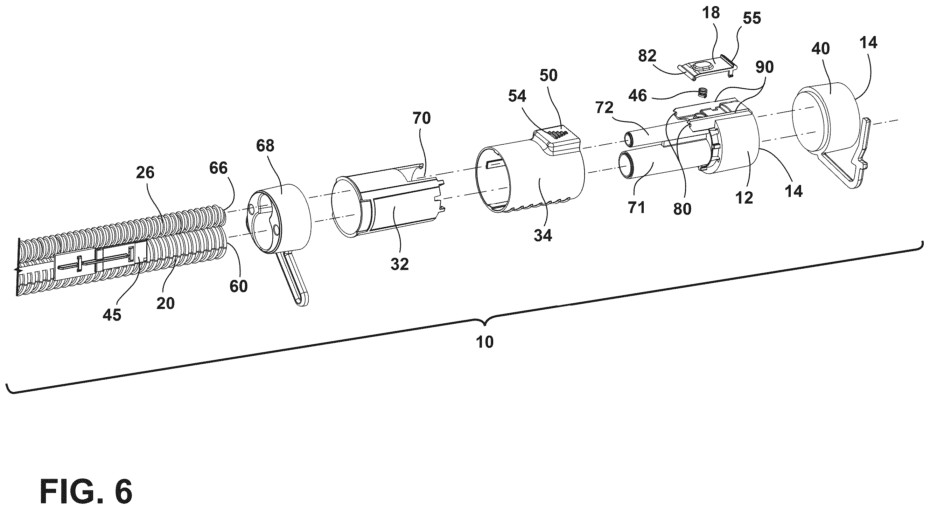

With reference now to FIGS. 3A and 3B, the hose connector assembly 10 is configured to engage with a connection feature 40 of the patient support apparatus 24. The connector housing 12 includes a generally circular configuration. The connection feature 40 includes an open end 42 configured to supply fluid and data to a controller that may be operably coupled to the patient support apparatus 24. An engagement assembly of the hose connector assembly 10 is configured to receive the latch 18 and secure the latch 18 to a receiving assembly of the patient support apparatus 24. A first tube 41 that defines a first outlet 41A and a second tube 43 that defines a second outlet 43A relay fluids to the connection feature 40, as explained in further detail below. Similarly, contacts 45 are disposed in the connector housing 12 and are in electrical communication with the data line 30. The contacts 45 constitute an exposed data interface at the interface opening 14 that is available for connection with the patient support interface 22. The latch 18 is operable between an engaged, or latched, position and a disengaged, or unlatched, position. Moreover, the latch 18 is spring-biased to the engaged position by a compression spring 46 (FIGS. 5 and 6). The data line 30 extends into the hose connector assembly 10 and terminates at contacts 45 disposed within the connector housing 12.

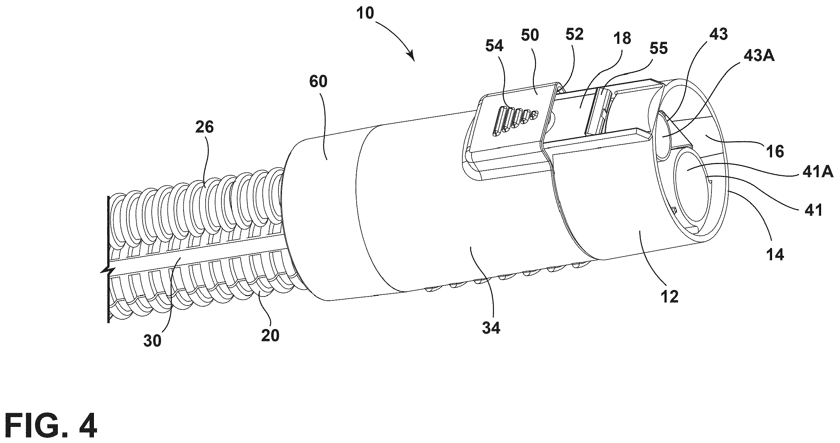

As further shown in FIGS. 3A, 3B, and 4, the latch 18 is disposed between the connector housing 12 and the top cover 34. The top cover 34 includes a protuberance 50 that defines a recess 52 that receives the latch 18. In some instances, the top cover 34 may include a gripping area 54 proximate the protuberance 50 for assisting a caregiver in removing or connecting the hose connector assembly 10 from the connection feature 40. The latch 18 includes a catch 55 disposed between the recess 52 and the interface opening 14.

With reference now to FIG. 5, the illustrated hose connector assembly 10 is configured to engage the first hose 20 and the second hose 26. The first hose 20 includes an internal tube 60 that defines a pathway through which fluids can flow. Similarly, the second hose 26 includes an internal tube 66 through which fluids can flow. A strain relief member 68 is disposed adjacent a proximal end of the hose connector assembly 10 and defines apertures 68A and 68B. The strain relief member 68 is configured to minimize stress where the first hose 20 and the second hose 26 engage the hose connector assembly 10. The bottom cover 32, which is slidingly received within the top cover 34, includes a slot 70 configured to provide space for the latch 18. The bottom cover 32 extends inside the top cover 34 and at the same time extends over a first tube 71 and a second tube 72 of the connector housing 12 The first tube 71 and the second tube 72 define first and second receiving ports, respectively. The first tube 71 is received within the internal tube 60 of the first hose 20. Likewise, the second tube 72 is received within the internal tube 66 of the second hose 26. This linear configuration results in linear flow of fluids through the internal tube 60 to the tube 71 and through the internal tube 66 to the second tube 72. This linear flow of fluids minimizes or eliminates noise and fluid loss of the entire hose connector assembly 10.

The latch 18 interfaces with a recesses 80 configured to receive a pivot member 82. The pivot member 82 is rotatable about a pivot axis defined by the recesses 80 that extends orthogonal to a longitudinal extent of the hose connector assembly 10. As previously noted, the latch 18 is spring-biased outward by the spring 46. Although a compression spring is illustrated, it is contemplated that the spring 46 may take on a variety of configurations. The latch 18 extends between receiving walls 90 disposed on either side of the latch 18. The latch 18, the receiving walls 90, and the pivot member 82 are all received within the protuberance 50.

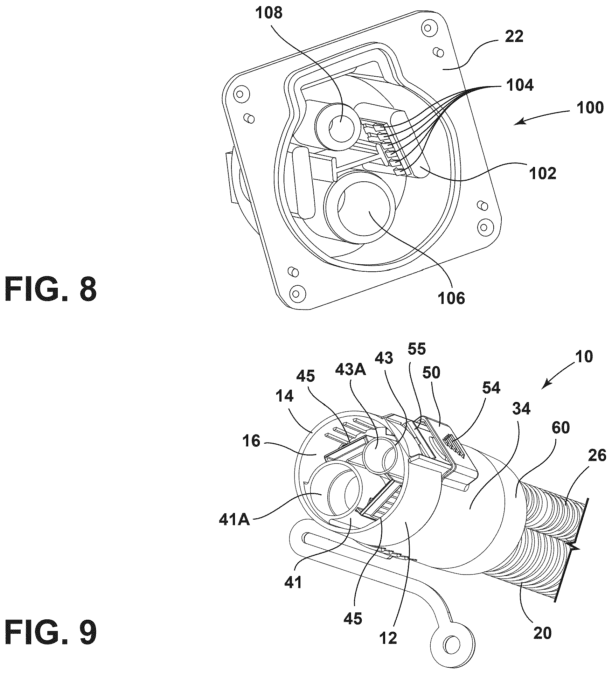

With reference now to FIGS. 8 and 9, the hose connector assembly 10 is configured to engage a receiving assembly 100 coupled to the patient support apparatus 24 via the patient support interface 22. The receiving assembly 100 includes an electrical interface 102 configured to engage the data line 30, and more specifically includes contacts 104 configured to engage the contacts 45 of the data line 30. A first receiving tube 106 is configured to engage the first outlet 41A of the hose connector assembly 10. Likewise, a second receiving tube 108 is configured to engage the second outlet 43A of the hose connector assembly 10. The data line 30 may transfer data collected from various detectors and sensors used to monitor a patient or user. This may include a variety of data, including vital signs like blood pressure, heart rate, and oxygen levels, as well as other health metrics such as blood sugar, weight, etc. The digital medical devices that are used to collect and electronically transmit this information to healthcare providers allows for remote and ongoing management of a patient's condition by a caregiver. The data line 30 may also convey operating parameters of a micro-climate management ("MCM") system to a caregiver to confirm proper use of the system and to adjust the operating parameters when appropriate. The data line 30 may track and adjust features of the MCM system and regulate operation of an air moving device operably coupled with the MCM system.

The hose connector assembly 10, as set forth herein, may deliver air from the air moving device (which may be a blower, fan, vacuum assembly, etc.) and compressor in a lower frame to a surface of the patient support apparatus 24 and, at the same time, provides an electrical connection from the lower frame to the surface of the patient support apparatus 24. The MCM system may be used to deliver airflow from the air moving device to the patient support apparatus 24. Airflow may also be delivered from the air moving device to the patient support apparatus 24 through use of a percussion and vibration system, and more specifically to a percussion and vibration valve during operation of the percussion and vibration system. The hose connector assembly 10 is configured to deliver air pressure from the compressor to support bladders, which may include turn assist bladders and other advanced articulation bladders, without significant pressure loss in the systems.

Airflow is relatively constant during operation of the MCM system. However, during operation of the percussion and vibration system, the airflow may be cycled on and off at a selected frequency, as deemed appropriate for the selected percussion and vibration therapy. The selected frequency often ranges from 1 Hz to 25 Hz, and results in an oscillating pressure through the hose connector assembly 10. In known constructions, to mitigate leakage, which may result in a pulsing noise being emitted from the hose connector assembly, epoxy has been utilized around the electrical cables. Consequently, a hose connector assembly design, such as the hose connector assembly 10 set forth herein, that allows linear airflow and minimal air leakage has substantial value. More specifically, the hose connector assembly 10, as set forth herein, makes direct contact with the first and second hoses 20, 26 that extend from the frame of the patient support apparatus 24 to eliminate air leaks and noise levels. This construction eliminates the need for epoxy, resin, and other sealing adhesives which are costly and can require additional labor. Consequently, this construction also improves the manufacturability of the part.

Elimination of epoxy, resin or other sealing adhesive minimizes the likelihood of shrinkage, improves the manufacturing process, and reduces the risk of cracking and brittleness of the hose connector assembly 10. The direct contact between the hose connector assembly 10 and the first and second hoses 20, 26 eliminates the need for a separate gasket or seal. This results in improved performance and reduces the risk of air leaks and noise. In addition, this structure achieves a tighter seal between components, thereby improving overall efficiency of the hose connector assembly 10 and provides a better linear flow of air, resulting in the compressor or pump working more efficiently and increasing overall longevity of the compressor, pump, and hose connector assembly.

Embodiments of the invention can be described with reference to the following numbered clauses, with preferred features laid out in the dependent clauses:
1. A hose connector assembly for a patient support apparatus, comprising:
   a connector housing defining an interface opening and a hose receiver;
   a latch operably coupled with the connector housing and spring-biased to a latched position;
   first and second hoses configured to engage with the hose receiver;
   a bottom cover extending over the first and second hoses and the hose receiver;
   a top cover extending over the bottom cover and at least partially concealing the latch, wherein the fluid is configured to pass through the first and second hoses in a substantially linear direction thereby minimizing noise and fluid loss of said hose connector assembly.
2. The hose connector assembly of clause 1, wherein the first and second hoses engage first and second receiving ports of the hose receiver.
3. The hose connector assembly of either one of clauses 1 or 2, wherein the first and
   second hoses include a barbed engagement end.
4. The hose connector assembly of any one of clauses 1-3, wherein the bottom cover includes a recess for receiving the latch.
5. The hose connector assembly of any one of clauses 1-4, further comprising:
   a strain relief member disposed proximate a distal end of the top cover.
6. The hose connector assembly of any one of clauses 1-5, further comprising:
   a data line proximate the first hose.
7. The hose connector assembly of clause 6, wherein the data line includes an exposed data interface at the interface opening.
8. The hose connector assembly of any one of clauses 1-7, wherein a diameter of the first hose and a diameter of the second hose differ.
9. A hose connector assembly, comprising:
   a connector housing defining an interface opening and a hose receiver;
   a latch operably coupled with the connector housing and spring-biased to a latched position;
   a first hose configured to engage with the hose receiver, the first hose supplying fluid to a patient support interface of a patient support apparatus;
   a second hose configured to engage with the hose receiver, the second hose being smaller in diameter than the first hose;
   a data line configured to engage a data interface of the patient support apparatus;
   a bottom cover extending over the first and second hoses and the hose receiver;
   a top cover extending over the bottom cover and at least partially concealing the latch, wherein the fluid is configured to pass through the first and second hoses in a substantially linear direction thereby minimizing noise and fluid loss of said hose connector assembly.
10. The hose connector assembly of clause 9, further comprising:
   a strain relief member disposed adjacent the top cover.
11. The hose connector assembly of clause 10, wherein the bottom cover is completely concealed by the top cover and the strain relief member.
12. The hose connector assembly of any one of clauses 9-11, wherein the top cover defines a recess that receives the latch.
13. The hose connector assembly of clause 12, wherein the top cover includes a gripping area proximate the recess.
14. The hose connector assembly of either one of clauses 12 or 13, wherein the latch includes a catch disposed between the recess and the interface opening.
15. A patient support apparatus, comprising:
   a patient support;
   a hose connector assembly, the hose connector assembly comprising:
      a connector housing defining an interface opening and a hose receiver;
      a latch operably coupled with the connector housing and spring-biased to a latched position;
      a first hose configured to engage with the hose receiver, the first hose supplying fluid to a patient support interface of said patient support apparatus;
      a second hose configured to engage with the hose receiver;
      a data line configured to engage a data interface disposed at the patient support; a
      top cover extending over a bottom cover, wherein the fluid is configured to pass through the first and second hoses in a substantially linear direction thereby minimizing noise and fluid loss of the hose connector assembly.
16. The patient support apparatus of clause 15, wherein the data line terminates at contacts disposed in the connector housing.
17. The patient support apparatus of either one of clauses 15 or 16, wherein the data line includes an exposed data interface at the interface opening.
18. The patient support apparatus of any one of clauses 15-17, wherein a diameter of the first hose and a diameter of the second hose differ.
19. The patient support apparatus of any one of clauses 15-18, wherein the top cover defines a recess that receives the latch.
20. The patient support apparatus of any one of clauses 15-19, wherein the first and second hoses engage first and second receiving ports of the hose receiver.

Other aspects of the present disclosure may be as follows:

In one aspect, a connector assembly for a patient support apparatus includes a connector housing that defines an interface opening and a hose receiver. A latch is operably coupled with the connector housing and is spring-biased to a latched position. First and second hoses are configured to engage with the hose receiver. A bottom cover extends over the first and second hoses and a portion of the hose receiver and a top cover extends over the bottom cover and at least partially conceals the latch. Fluid is configured to pass through the first and second hoses into the connector housing in a substantially linear direction thereby minimizing noise and fluid loss of the connector assembly.

According to still another aspect of the present disclosure, first and second hoses are in fluid communication with first and second receiving ports of a hose receiver.

According to another aspect of the present disclosure, first and second hoses include a barbed engagement end.

According to yet another aspect of the present disclosure, a bottom cover includes a recess for receiving a latch.

According to another aspect of the present disclosure, a hose connector assembly includes a strain relief member disposed adjacent a proximal end of the hose connector assembly and defines apertures.

According to still another aspect of the present disclosure, a hose connector assembly includes a data line that is proximate a first hose.

According to another aspect of the present disclosure, a data line includes an exposed data interface at an interface opening.

According to yet another aspect of the present disclosure, a diameter of a first hose and a diameter of a second hose differ.

According to another aspect of the present disclosure, a hose connector assembly includes a connector housing that defines an interface opening and a hose receiver. A latch is operably coupled with the connector housing and is spring-biased to a latched position. A first hose is configured to engage with the hose receiver and supplies fluid to a patient support interface of a patient support apparatus. A second hose is configured to engage with the hose receiver and is smaller in diameter than the first hose. A data line is configured to engage a data interface of the patient support apparatus. A bottom cover extends over the first and second hoses and the hose receiver and a top cover extends over the bottom cover and at least partially conceals the latch. The fluid is configured to pass through the first and second hoses and through the connector housing in a linear direction thereby minimizing noise and fluid loss of the hose connector assembly.

According to still another aspect of the present disclosure, a hose connector assembly includes a strain relief member disposed adjacent to a top cover.

According to another aspect of the present disclosure, a bottom cover is completely concealed by a top cover and a strain relief member.

According to yet another aspect of the present disclosure, a top cover defines a recess that receives a latch.

According to another aspect of the present disclosure, a top cover includes a gripping area proximate to a recess.

According to another aspect of the present disclosure, a patient support apparatus includes a patient support and a hose connector assembly. The hose connector assembly includes a connector housing that defines an interface opening and a hose receiver. A latch is operably coupled with the connector housing and is spring-biased to a latched position. A first hose is configured to engage with the hose receiver and supplies fluid to a patient support interface of the patient support apparatus. A second hose is configured to engage with the hose receiver. A data line is configured to engage a data interface disposed at the patient support. A top cover extends over a bottom cover. The fluid is configured to transition from the first and second hoses to the hose connector assembly in a linear direction thereby minimizing noise and fluid loss of the hose connector assembly.

According to yet another aspect of the present disclosure, a latch includes a catch disposed between a recess and an interface opening.

According to still another aspect of the present disclosure, a data line terminates at contacts disposed in a connector housing.

It will be understood by one having ordinary skill in the art that construction of the described disclosure and other components is not limited to any specific material. Other exemplary embodiments of the disclosure disclosed herein may be formed from a wide variety of materials, unless described otherwise herein.

For purposes of this disclosure, the term "coupled" (in all of its forms, couple, coupling, coupled, etc.) generally means the joining of two components (electrical or mechanical) directly or indirectly to one another. Such joining may be stationary in nature or movable in nature. Such joining may be achieved with the two components (electrical or mechanical) and any additional intermediate members being integrally formed as a single unitary body with one another or with the two components. Such joining may be permanent in nature or may be removable or releasable in nature unless otherwise stated.

It is also important to note that the construction and arrangement of the elements of the disclosure, as shown in the exemplary embodiments, is illustrative only. Although only a few embodiments of the present innovations have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recited. For example, elements shown as integrally formed may be constructed of multiple parts, or elements shown as multiple parts may be integrally formed, the operation of the interfaces may be reversed or otherwise varied, the length or width of the structures and/or members or connector or other elements of the system may be varied, the nature or number of adjustment positions provided between the elements may be varied. It should be noted that the elements and/or assemblies of the system may be constructed from any of a wide variety of materials that provide sufficient strength or durability, in any of a wide variety of colors, textures, and combinations. Accordingly, all such modifications are intended to be included within the scope of the present innovations. Other substitutions, modifications, changes, and omissions may be made in the design, operating conditions, and arrangement of the desired and other exemplary embodiments without departing from the spirit of the present innovations.

It will be understood that any described processes or steps within described processes may be combined with other disclosed processes or steps to form structures within the scope of the present disclosure. The exemplary structures and processes disclosed herein are for illustrative purposes and are not to be construed as limiting.

## Claims

1. A hose connector assembly (10) for a patient support apparatus (24), comprising:
a connector housing (12) defining an interface opening (14) and a hose receiver (16);
a latch (18) operably coupled with the connector housing (12) and spring-biased to a latched position;
first and second hoses (20, 26) configured to engage with the hose receiver (16);
a bottom cover (32) extending over the first and second hoses (20, 26) and a portion of the hose receiver (16);
a top cover (34) extending over the bottom cover (32) and at least partially concealing the latch (18), wherein fluid is configured to pass through the first and second hoses (20, 26) into the connector housing (12) in a substantially linear direction thereby minimizing noise and fluid loss of said hose connector assembly (10).

2. The hose connector assembly (10) of claim 1, wherein the first and second hoses (20, 26) are in fluid communication with first and second receiving ports of the hose receiver (16).

3. The hose connector assembly (10) of either one of claims 1 or 2, wherein the first and second hoses (20, 26) include a barbed engagement end.

4. The hose connector assembly (10) of any one of claims 1-3, wherein the top cover (34) defines a recess (52) for receiving the latch (18).

5. The hose connector assembly (10) of any one of claims 1-4, further comprising:
a strain relief member (68) disposed adjacent a proximal end of said hose connector assembly (10) and defines apertures (68A, 68B).

6. The hose connector assembly (10) of any one of claims 1-5, further comprising:
a data line (30) proximate the first hose (20).

7. The hose connector assembly (10) of claim 6, wherein the data line (30) includes an exposed data interface at the interface opening (14).

8. The hose connector assembly (10) of any one of claims 1-7, wherein a diameter of the first hose (20) and a diameter of the second hose (26) differ.

9. The hose connector assembly (10) of claim 5, wherein the bottom cover (32) is completely concealed by the top cover (34) and the strain relief member (68).

10. The hose connector assembly (10) of claim 4, wherein the top cover (34) includes a gripping area (54) proximate the recess (52).

11. The hose connector assembly (10) of either one of claims 4 or 10, wherein the latch (18) includes a catch (55) disposed between the recess (52) and the interface opening (14).

12. The hose connector assembly (10) of claim 6, wherein the data line (30) terminates at contacts (45) disposed in the connector housing (12).

13. The hose connector assembly (10) of either one of claims 6 or 12, wherein the data line (30) may track and adjust features of a micro-climate management ("MCM") system and regulate operation of an air moving device.

14. The hose connector assembly (10) of any one of claims 1-13, wherein said hose connector assembly (10) is free of adhesives.

15. The hose connector assembly (10) of any one of claims 1-14, wherein all or a majority of at least one of the first hose 20 and the second hose 26 are corrugated at a circumference thereof.
